(19) Europäisches Patentamt
European Patent Office
Office européen des brevets

(11) Publication number: **0 081 782**
**B1**

(12) **EUROPEAN PATENT SPECIFICATION**

(45) Date of publication of patent specification: **30.10.85**

(21) Application number: **82111258.8**

(22) Date of filing: **06.12.82**

(51) Int. Cl.[4]: **C 07 C 93/14**, C 07 C 91/30, C 07 C 103/44, C 07 D 285/10, C 07 D 213/74, C 07 D 215/38, A 61 K 31/135, A 61 K 31/41, A 61 K 31/445, A 61 K 31/47

(54) **Hydroxybenzylamino-aryl compounds, process for preparing and pharmaceutical compositions containing the same.**

(30) Priority: **14.12.81 US 330542**

(43) Date of publication of application:
**22.06.83 Bulletin 83/25**

(45) Publication of the grant of the patent:
**30.10.85 Bulletin 85/44**

(84) Designated Contracting States:
**AT BE CH DE FR GB IT LI LU NL SE**

(56) References cited:
**US-A-2 784 138**
**US-A-3 996 278**

**CHEMICAL ABSTRACTS, vol. 86, no. 9, 28th February 1977, page 366, no. 54830q, Columbus Ohio (USA); J. STRADINS et al.: "Comparative voltammetric study of para-substituted phenols and their N-phenylaminomethyl derivatives of a graphite anode".**

(73) Proprietor: **MERCK & CO. INC.**
**126, East Lincoln Avenue P.O. Box 2000**
**Rahway New Jersey 07065 (US)**

(72) Inventor: **Jensen, Norman P.**
**5 Woodcrest Drive**
**New Providence New Jersey (US)**
Inventor: **Chang, Michael N.**
**746 Austin Street**
**Westfield New Jersey (US)**

(74) Representative: **Abitz, Walter, Dr.-Ing. et al**
**Abitz, Morf, Gritschneder, Freiherr von Wittgenstein Postfach 86 01 09**
**D-8000 München 86 (DE)**

(56) References cited:
**ARCHIV DER PHARMAZIE, vol. 298, no. 7, 1965, pages 423-434, Weinheim (DE); R.POHLOUDEK-FABINI et al.: "Verhalten rhodanierter Schiffscher Basen gegenüber Raney-Nickel, Hydrazin und Alkalien".**

**EP 0 081 782 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Background of the Invention

The present invention relates to hydroxybenzylaminoaryl compounds (some of which are novel) useful as topical anti-inflammatory agents. We have found that the compounds are active in both the peritoneal macrophage assay and the U.V. erythema assay for topical anti-inflammatory agents. However, these compounds are rapidly inactivated *in vivo* and devoid of any systemic effects.

Recent studies demonstrated that macrophages participate in the development and progression of chronic inflammatory diseases such as rheumatoid arthritis. During the progression of inflammatory conditions, there is generally an appearance and/or presence of macrophages and lymphocytes, especially macrophages and polymorphonuclear leukocytes. Macrophages are known to secrete various products in response to inflammatory stimuli. For example:

1) Neutral proteinases — the destructive peptide bond cleaving enzyme which has been shown to be directly involved in rheumatoid cartilage destruction; and

2) Prostaglandins (PG) (e.g., $E_2$ and $I_2$ by mouse peritoneal macrophages) and other arachiodonic acid derivatives derived from both the cyclooxygenase and the lipoxygenase pathways.

These arachiodonic acid oxygenation products have been identified as the critical mediators of various acute inflammatory conditions.

Accordingly, pharmacological agents which are capable of inhibiting the formation, the release, or the function of macrophages may also be effective agents in the treatment of rheumatoid arthritis, emphysema, bronchial inflammation, ostearthritis, psonasis, acute respiratory distress syndrome, spondylistis, lupus, gout and other inflammatory diseases.

With respect to the U.V. erthema assay, it has been shown previously that the U.V. erthema condition is partially the result of a local release of prostaglandins derived oxidatively from arachiodonic acid by the action of PG synthetases, e.g., cyclooxygenase and lipooxygenase. Therefore, pharmacological agents which inhibit the erythema are generally considered to be active topical anti-inflammatory agents.

Furthermore, anti-inflammatory agents which are not systemically active are advantageous in the sense that they are not subject to the adverse effects, e.g., gastrointestinal ulcerations and bleeding that often plagued users of systemic NSAIAs (non-steroidal anti-inflammatory agents). Accordingly, an object of this invention is to provide novel hydroxybenzylaminoaryl compounds as topical anti-inflammatory agents useful in the treatment of dermal inflammatory conditions and prusitus such as sunburn, erythema, eczema, contact dermatitis, and allergic dermatitis, and psoriasis.

Another object of this invention is to provide appropriate processes for the preparation of the subject novel compounds.

Still a further object of the present invention is to provide a pharmaceutically acceptable composition containing an effective amount of the active compound for the treatment of various dermatological inflammatory conditions.

Detailed Description of the Invention

This invention relates to novel derivatives of hydroxybenzylamine of the structural formula (I):

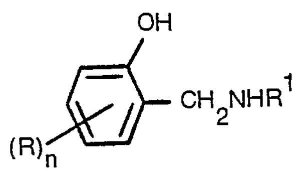

(I)

or a pharmaceutically acceptable salt thereof.

Wherein

R is

(a) fluoro, except that when n is 2, the two fluoro groups cannot be at position 3 and 5 respectively;

(b) methoxy, except that when n is 1, the methoxy group cannot be at position 3, ethoxy, n-propoxy or i-propoxy;

(c) methylthio, ethylthio, n-propylthio or i-propylthio;

(d) $-O-CH_2-O-$;

(e) $-COOH$; or

(f) aryloxy especially phenoxy;

$R^1$ is

(a) unsubstituted or substituted phenyl especially phenyl, 3-aminophenyl, 4-(3,3-dimethylbutyryl-amino)phenyl, 3-methylphenyl, 3,5-dimethoxyphenyl, 3-ethylthiophenyl, 4-chlorophenyl, 4-trifluoromethylphenyl, 2,4-difluorophenyl, 4-nitrophenyl, except that when R is fluoro, $R^1$ cannot be a halosubstituted phenyl; or

(b) unsubstituted or substituted heteroaryl especially monoheteroaryl such as thiadiazolyl, pyrryl, pyridyl or pyrazinyl, imidazolyl, thiaimidazolyl, quinolino or tetrazolyl; the heteroaryl can for example be substituted with one or more of $C_{1-6}$ alkyl, $C_{1-6}$ alkoxy, $C_{1-6}$ alkylthio, halo especially chloro or fluoro

n is 1 or 2;

with the proviso that the compounds of structural formula (I) cannot be N-(2-hydroxy-5-methoxy-benzyl)-aniline.

In the most preferred embodiment of this invention,

R is methoxy, ethoxy or isopropoxy; or methylthio, ethylthio or n- or i-propylthio;

$R^1$ is unsubstituted or substituted phenyl.

The novel compounds of the present invention are prepared from a process comprising:

*Step (1):* treating an appropriately substituted 2-hydroxy-benzaldehyde of the structural formula (II)

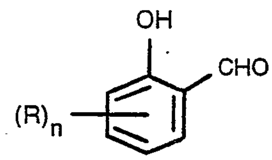

(II)

with an amine of formula $R^1NH_2$ to form a Schiff-base; and

*Step (2):* treating the resulting Schiff-base with a reducing agent such as hydrogen in the presence of a catalyst, e.g., Pd/C or sodium tetrahydroboron.

In Step (1), the Schiff-base formation is well-documented. See M. M. Sprung, *Chem. Rev., 26,* 297 (1940); R. W. Layer, *Chem. Rev., 63,* 489 (1963); and G. Hilgetag and A. Martini, *Preparative Organic Chemistry,* Wiley-Interscience, pp. 504—508 (1968). Generally, the benzaldehyde is treated with neat amine or a solution thereof at relatively low temperatures, e.g., from about 0°C to about 25°C with adequate stirring until the reaction is substantially complete. The solution of the amine is usually prepared by dissolving the amine in an organic solvent preferably water, lower alcohol such as methanol, ethanol, isopropyl alcohol or an aqueous solution thereof. Under optimum conditions the reaction usually takes from about 1 to about 24 hours.

In most cases, Step (2) can be conveniently merged into Step (1) by the simple addition of a reducing agent to the reaction mixture of step (1). The crude Schiff-base in the reaction mixture is thus reduced *in situ* to the desired hydroxybenzylamino derivatives of formula (I).

The most commonly used reducing agents, among others, are hydrogen in the presence of Pd/C and sodium borohydride. Other metal hydrides, for example, lithium borohydride, lithium aluminumhydride $NaCNBH_3$ and other substituted aluminum or borohydrides may also be used. See H. O. House, *Modern Synthetic Reactions,* 2nd ed., W. A. Benjamin, Inc., 1972, pp. 45—54.

The reduction is usually conducted at relatively low temperatures, e.g., from about 0°C to about 30°C, preferably below 25°C. A solvent is usually required. The commonly used solvents for each reducing agent are summarized below in Table I.

TABLE I

| Reducing Agent | Useful reaction solvents |
|---|---|
| $NaBH_4$ | $H_2O$, $CH_3OH$, EtOH, i-PrOH, diglyme |
| $LiBH_4$ | Tetrahydrofuran, diglyme |
| $LiAlH_4$ | Diethylether, tetrahydrofuran, 1,2-dimethoxyethane, and diglyme |
| $H_2$/Pd/C | Ethylacetate, ethanol, water, acetic acid |

Usually the reduction is substantially complete within from about 1 hour to about 48 hours. However, under optimum conditions, the reaction may take only 1 to 6 hours.

The starting materials in the preparation of the novel hydroxybenzylamine derivatives are mostly commercially available or can be easily prepared *via* conventional methods, for example, 4-(3,3-dimethylbutyrylamino)aniline is simply derived from the reaction between 4-nitro-aniline and 3,3-dimethylbutyryl chloride followed by hydrogenation.

As the novel compounds of this invention are organic bases, their pharmaceutically acceptable salts are those resulting from the neutralization of the base with an acid. The acid employed is usually an inorganic acid such as a hydrohalic acid, e.g., hydrochloric or hydrobromic acid; sulfuric acid; nitric acid; or

phosphoric acid. An organic acid such as maleic, fumaric, tartaric, citric, acetic, salicyclic, succinic, benzoic, benzenesulfonic, glutamic, lactic or isethionic acid is also commonly used. Generally the neutralization is conducted in an inert solvent such as water; a $C_{1-3}$ alkanol such as methanol, ethanol or isopropanol; a $C_{3-6}$-ketone such as acetone, or ethylmethyl ketone; an ethereal solvent such as diethyl ether, tetrahydrofuran or dioxane; acetonitrile; or an aromatic solvent such as toluene. Mixtures of the above described solvents are also employed. Generally the neutralization is carried out in aqueous ethanol, at 0°—75°C, preferably at 0°—25°C, followed by filtration to collect the salts.

This invention also relates to compositions for treating topical inflammation in patients in need of such treatment. Generally, a sufficient amount of a compound of formula (I) or a pharmaceutical composition thereof, particularly an especially preferred compound, is administered to the patient as the active constituent.

The U.V. erythema assay by which topical anti-inflammatory activity is determined, is based on the ability of the compounds of Formula I to inhibit the erythema induced on the skin of guinea pigs by UV radiation. It has been substantiated by recent studies that prostaglandin synthesis and the release thereof may be the primary process involved in the production of erythema. A protocol of the assay and some results thereof are summarized below in table II.

TABLE II

U.V. Erythema Assay

*Procedure*

Guinea pigs were depilated and stabilized for 30 minutes. Following anesthesia with Nembutal (35 mg/kg), they were exposed to a U.V. lamp for 45 minutes at a distance of seven inches from the abdominal surface. Compound was applied topically (seven applications in a total of 0.1 ml vehicle) 15 minutes post exposure. The surface was then gently washed and read two hours post treatment.

*Vehicle 1774* — 85% EtOH, 12% Propylene Glycol, 3% Methyl Salicylate

Erythema Scoring

| Designation | Numerical Score | Description |
|---|---|---|
| Negative | 0 | No erythema (normal skin color) |
| Trace | +1 | Faint pink in some areas |
| Slight | +2 | Faint pink over entire site |
| Slight-Moderate | +3 | Faint pink to red |
| Moderate | +4 | Red |
| Marked | +5 | Red to purplish red |

*Evaluation of Erythema*

A numerical value 0—5 was assigned to the degree of erythema observed under standard lighting conditions. Drug effect on the developing erythema was calculated as follows:

$$\% \text{ suppression} = \left( \frac{\text{(score of vehicle treated)} - \text{(Score of drug Treated)}}{\text{score of vehicle treated}} \right) \times 100$$

*Results:* % Inhibition of Erythema by N-(2-hydroxy-5-methoxybenzyl)aniline

| Dosage | % Inhibition (mean of 3 animals) | | | | |
|---|---|---|---|---|---|
| (mg) | 2 Hr. | 3 Hr. | 4 Hr. | 6 Hr. | 24 Hr. |
| 0.1 | 19.3 | 16.7 | 15.0 | 6.7 | 0 |
| 0.3 | 55.3 | 50.0 | 46.7 | 35.0 | 0 |
| 1.0 | 80.3 | 91.7 | 76.7 | 63.3 | 26.7 |
| 3.0 | 100 | 100 | 63.3 | 43.3 | 33.3 |

For treatment of inflammation, fever or pain, the compounds of the invention are administered topically, by inhalation spray or rectally in dosage unit formulations containing conventional non-toxic pharmaceutically acceptable carriers, adjuvants and vehicles. In addition to the treatment of warm-blooded animals such as mice, rats, horses, dogs, cats, etc., the compounds of the invention are effective in the treatment of humans.

The pharmaceutical compositions containing the active ingredient may be in a form suitable for topical use, for example, aqueous or oily solutions or suspensions, dispersible powders or granules, tinctures, topical aerosol emulsions, creams, ointments, jellies, suppositories or the like. Compositions intended for topical use may be prepared according to any method known to the art for the manufacture of pharmaceutical compositions and such compositions may contain one or more active compounds.

Aqueous suspensions contain the active materials in admixture with excipients suitable for the manufacture of aqueous suspensions. Such excipients are suspending agents, for example sodium carboxymethylcellulose, methylcellulose, hydroxypropylmethylcellulose, sodium alginate, polyvinylpyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyoxyethylene sorbitan monooleate. The said aqueous suspensions may also contain one or more preservatives, for example, ethyl, or n-propyl p-hydroxybenzoate.

Oily suspension may be formulated by suspending the active ingredient in a vegetable oil, for example, arachis oil, olive oil, sesame oil or coconut oil, or in a mineral oil such as liquid paraffin. The oily suspensions may contain a thickening agent, for example, beeswax, hard paraffin or cetyl alcohol. These compositions may be preserved by the addition of an antioxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above.

The pharmaceutical compositions of the invention may also be in the form of oil-in-water emulsions. The oily phase may be a vegetable oil, for example, olive oil or arachis oils, or a mineral oil, for example, liquid paraffin or mixtures of these. Suitable emulsifying agents may be naturally-occurring gums, for example, gum acacia or gum tragacanth, naturally-occurring phosphatides, for example, soy bean, lecithin, and esters or partial esters derived from fatty acids and hexitol anhydrides, for example, sorbitan monooleate, and condensation products of the said partial esters with ethylene oxide, for example, polyoxyethylene sorbitan monooleate.

An ointment containing the pharmaceutical compositions of the present invention may be prepared, among other methods known in the art, by combining the active ingredient with a medium consisting of a glycol, a lower alkanol, and water; a gelling agent; and optionally an adjuvant such as diisopropyl adipate, diethyl sebacate, ethyl carproate and ethyl laurate. Suitable glycols include propylene glycol, butylene glycol, polyethylene glycol and the like. Generally, a carboxyvinyl polymer preneutralized with an organic amine such as diisopropyl amine and triethylamine, or a cellulose, e.g., hydroxyethyl cellulose, methyl cellulose, carboxymethyl cellulose, hydroxypropyl cellulose, is used as the gelling agent.

The compounds of the invention may also be administered in the form of suppositories for rectal administration of the drug. These compositions can be prepared by mixing the drug with a suitable nonirritating excipient which is solid at ordinary temperatures but liquid at the rectal temperature and will therefore melt in the rectum to release the drug. Such materials are cocoa butter and polyethylene glycols.

Dosage levels of the order to 0.2 mg to 140 mg per kilogram of body weight per day are useful in the treatment of the above-indicated conditions (10 mg to 7 gms per patient per day). For example, inflammation is effectively treated by the administration from about 0.5 to 50 mg of the compound per kilogram of body weight per day (25 mg to 5 gms per patient per day). Advantageously, from about 2 mg to about 20 mg per kilogram of body weight per daily dosage produces highly effective results (50 mg to 1 gm per patient per day).

The amount of active ingredient that may be combined with the carrier materials to produce a single dosage form will vary depending upon the host treated and the particular mode of administration. Dosage unit forms will generally contain between from about 25 mg to about 1 g of active ingredient.

It will be understood, however, that the specific dose level for any particular patient will depend upon a variety of factors including the activity of the specific compound employed, the age, body weight, general health, sex, diet, time of administration, route of administration, rate of excretion, drug combination and the severity of the particular disease undergoing therapy.

Example 1

4-(2-hydroxy-5-methoxybenzlamino)-3,3-dimethylbutyrylaminobenzene

*Step A: Preparation of (3,3-dimethylbutyrylamino)aniline*

Five grams of 4-nitroaniline was stirred in 25 ml of pyridine and 4.95 g of 3,3-dimethylbutyryl chloride

was added. The mixture was stirred 15 hours and poured into ice-water. The resultant solid was collected on a filter and dissolved in 200 ml of ethyl acetate and the column was treated with activated charcoal and concentrated to yield 6.3 g of 4-nitro-3,3-dimethylbutyrylaminobenzene, m.p. 192—194°. This material was reduced in 200 ml of methanol, at 3 atmospheres of hydrogen in the presence of 5% Pd/carbon. After removal of the catalyst by filtration, concentration of the filtrate gives 5.3 g of product (3,3-dimethylbutyrylamino)aniline, m.p. 138—140°C.

*Step B: Preparation of 4-(2-hydroxy-5-methoxybenzylamino)-3,3-dimethylbutyrylamino benzene*

A mixture of 2.5 g of 3,3-dimethylbutyrylaminoaniline and 1.84 g of 2-hydroxy-5-methoxybenzaldehyde was stirred in 50 ml of methanol, under a nitrogen atmosphere, for 15 hours. The mixture was then refluxed until tlc indicates that most of the starting material had reacted. After cooling, 0.50 g of $NaBH_4$ was added in two batches, and the mixture was stirred 2.5 hours. After concentration 6.9 g of solid was collected which was triturated with 100 ml of water and enough 2.5N HCl to neutralize the supernatant. 3.95 g of crude product was collected and purified by treatment with activated charcoal and recrystallization from ethyl acetate to give 2.5 g of 4-(2-hydroxy-5-methoxybenzylamino)-3,3-dimethylbutyrylamino benzene, m.p. 126—129°C.

Following substantially the same procedure as described above but substituting for the starting materials used therein 5-(t-butyl)-2-hydroxy-3-iodobenzaldehyde and 4-acetaminoaniline, there was prepared 4-(5-t-butyl-2-hydroxy-3-iodobenzylamino)acetanilide. M.p. 134.5—136°C.

Example 2

3-(3,5-Dichloro-2-Hydroxybenzylamino)[1,2,5]thiadiazole

A mixture of 6.9 g of 3,5-dichloro-2-hydroxybenzaldehyde and 3-amino[1,2,5]thiazole hydrochloride was heated at 80° for 18 hours. After cooling, 6.9 g of precipitate, m.p. 174—175° was collected. This precipitate was added to 100 ml of methanol and the mixture was cooled in an ice-bath before 1.4 g of $NaBH_4$ is added. The mixture was stirred ½ hour and concentrated to give 9.0 g of solid which was purified by trituration with water and enough 2.5N HCl to give a neutral supernatant. The insolubles were then collected, heated with charcoal and recrystallized from methylene chloride to give 5.1 g of 3-(3,5-dichloro-2-hydroxybenzylamino)[1,2,5]thiadiazole. M.p. 131—132°C.

Example 3

N-(2-Hydroxy-5-Methoxybenzyl)Aniline

A mixture of 15.2 g 2-hydroxy-5-methoxy benzaldehyde, 93 g of aniline and 100 ml of methanol was stirred at 25° for 20 hours. The mixture was then cooled with an ice-bath and stirred, and 3.9 g of sodium borohydride was added slowly. The mixture was stirred for 1 hour at 25°, 150 ml of water was added and the pH was adjusted to 7.0 with 2.5N hydrochloric acid. The resultant solid was collected, washed with water and purified by chromatography on silica gel using 3:17 ethyl acetate:n-hexane as an eluant. A 17.7 g yield of N-(2-hydroxy-5-methoxybenzyl)aniline was obtained. M.p. 82—84°.

Example 4

N-(2-Hydroxy-5-methoxybenzyl)Aniline

A mixture of 15.2 g of 2-hydroxy-5-methoxybenzaldehyde, 15 g of aniline and 200 ml of methanol was stirred for 3 hours. The mixture was then hydrogenated at $2.76 \times 10^5$ Pa (40 psi) in the presence of 2.0 g of 10% Pd/C until 0.1 mole of hydrogen was consumed. The catalyst was removed by filtration and the filtrate was concentrated *in vacuo* to a yellow oil which was crystallized from ether-n-hexane to yield 18 g of N-(2-hydroxy-5-methoxybenzyl)aniline. M.p. 82.5—83°C.

Following substantially the same procedure as described above, but substituting for aniline used therein, the following compounds:

(1) 2,6-dichloroaniline
(2) 3,5-dimethylaniline
(3) 3,5-dimethoxyaniline
(4) 4-trifluoromethylaniline

There are obtained the following corresponding hydroxybenzylaminobenzene derivatives:

(1) 2,6-dichloro-N-(2-hydroxy-5-methoxybenzyl)aniline, m.p.
(2) 3,5-dimethyl-N-(2-hydroxy-5-methoxybenzyl)aniline
(3) 3,5-dimethoxy-N-(2-hydroxy-5-methoxybenzyl)aniline
(4) 4-trifluoromethylaniline

Example 5

Following substantially the same procedure as described in Example 4, but substituting for aniline used therein the following heterocyclic amines:

(1) 3-aminoquinoline
(2) 2-amino-4,6-dimethylpyridine

There were obtained the corresponding hydroxybenzyl amine derivatives as listed below:

(1) 3-[N-(2-hydroxy-5-methoxybenzyl)amino]quinoline, m.p. 101—103°C.

(2) 2-[N-(2-hydroxy-5-methoxybenzyl)amino]-4,6-dimethylpyridine m.p. 95—96°C.

Example 6

Set forth below are some illustrative topical formulations containing a selected active compound of the instant invention.

Formulation Number 1 — Solution

3-(3,5-dichloro-2-hydroxybenzylamino)[1,2,5]thiadiazole (a) — 2.5%

Distilled water qs to 100%

Procedure: Dissolve compound (a) in enough water to make 100%. Filter the solution. Apply to the affected area.

Formulation Number 2 — Topical Aerosol

N-(2-hydroxy-5-methoxybenzyl)Aniline (c) — 2.5%

Alcohol U.S.P. — 5%

Isopropylmyristate — 5%

Conventional halogenated hydrocarbon propellant qs 100% e.g., Freon 11 (trichlorofluoromethane), Freon 12 (dichlorodifluoromethane), Freon 14 (carbon tetrafluoride), Freon C 318 (Octafluorocyclobutane), Freon 114 (Cryofluorane), etc.

Procedure: Dissolve Compound (c) in the alcohol and isopropylmyristate. Add sufficient halogenated propellant and introduce into conventional aerosol containers either by pressure or by cold filing. Apply to affected area.

Formulation Number 3 — Ointment

4-(2-hydroxy-5-methoxybenzylamino)-3,3-dimethylbutyrylaminobenzene (d) — 2.5%

Petrolatum U.S.P. qs to 100%

Procedure: Heat the petrolatum to 60°C. Add compound (d) and stir until thoroughly dispersed. Cool to room temperature. Apply to affected area.

**Claims for the Contracting States: BE CH DE FR GB IT LI LU NL SE**

1. A compound of structural formula (I)

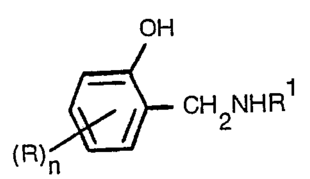

(I)

wherein:

R is

(a) fluoro, except that when n is 2, the two fluoro groups cannot be at positions 3 and 5 respectively;

(b) methoxy, except that when n is 1, the methoxy group cannot be at position 3; ethoxy, n-propoxy or i-propoxy;

(c) methylthio, ethylthio, n-propylthio or i-propylthio,

(d) $-OCH_2-O-$;

(e) —COOH; or

(f) aryloxy;

$R^1$ is

(a) unsubstituted or substituted phenyl except that when R is fluoro, $R^1$ cannot be a halo-substituted phenyl; or

(b) unsubstituted or substituted heteroaryl;

n is 1 or 2;

with the proviso that the compound of structural formula (I) cannot be N-(2-hydroxy-5-methoxybenzyl)aniline;

or a pharmaceutically acceptable salt thereof.

2. A process for the preparation of a compound of structural formula (I) according to Claim 1 comprising

(a) treating an appropriately substituted 2-hydroxybenzaldehyde of the structural formula II

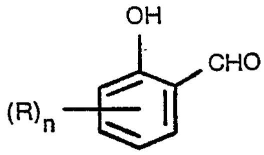

(II)

with an amine of formula $R^1NH_2$ to form a Schiff-base; and

(b) treating the Schiff-base with a reducing agent,

and optionally neutralizing the product obtained to form the pharmaceutically acceptable salt.

3. A pharmaceutical composition for treatment of topical inflammation comprising a pharmaceutical carrier and an effective amount of a compound of formula (I) according to Claim 1 and/or N-(2-hydroxy-5-methoxybenzyl)aniline.

4. The pharmaceutical composition of Claim 3 wherein the compound is N-(2-hydroxy-5-methoxybenzyl)aniline.

**Claim for the Contracting State: AT**

A process for the preparation of a compound of structural formula (I)

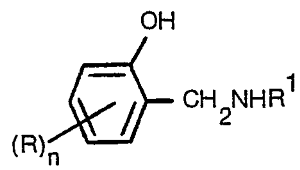

(I)

wherein:

R is

(a) fluoro, except that when n is 2, the two fluoro groups cannot be at positions 3 and 5 respectively;

(b) methoxy, except that when n is 1, the methoxy group cannot be at position 3; ethoxy, n-propoxy or i-propoxy;

(c) methylthio, ethylthio, n-propylthio or i-propylthio,

(d) $—OCH_2—O—$;

(e) —COOH; or

(f) aryloxy;

$R^1$ is

(a) unsubstituted or substituted phenyl except that when R is fluoro, $R^1$ cannot be a halo-substituted phenyl; or

(b) unsubstituted or substituted heteroaryl;

n is 1 or 2;

with the proviso that the compound of structural formula (I) cannot be N-(2-hydroxy-5-methoxybenzyl)aniline;

and the pharmaceutically acceptable salt thereof, comprising:

(a) treating an appropriately substituted 2-hydroxybenzaldehyde of the structural formula II

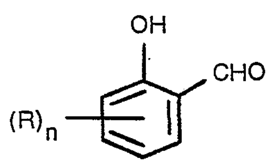

(II)

with an amine of formula $R^1NH_2$ to form a Schiff-base; and

(b) treating the Schiff-base with a reducing agent;

and optionally neutralizing the product obtained to form the pharmaceutically acceptable salt.

**Patentansprüche für die Vertragsstaaten: BE CH DE FR GB IT LI LU NL SE**

1. Eine Verbindung der Strukturformel (I)

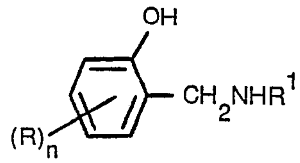

(I)

worin

R

(a) Fluor, mit der Ausnahme, daß, falls n 2 ist, die zwei Fluorgruppen nicht in Stellung 3 bzw. 5 vorliegen können;

(b) Methoxy, mit der Ausnahme, daß, falls n 1 ist, die Methoxygruppe nicht in Stellung 3 vorliegen kann; Ethoxy, n-Propoxy oder i-Propoxy;

(c) Methylthio, Ethylthio, n-Propylthio oder i-Propylthio;

(d) $—OCH_2—O—$;

(e) —COOH; oder

(f) Aryloxy bedeutet;

$R^1$

(a) unsubstituiertes oder substituiertes Phenyl, mit der Ausnahme, daß, falls R Fluor ist, $R^1$ nicht ein halogensubstituiertes Phenyl sein kann; oder

(b) unsubstituiertes oder substituiertes Heteroaryl bedeutet; und

n 1 oder 2 ist;

mit der Maßgabe, daß die Verbindung der Strukturformel (I) nicht N-(2-Hydroxy-5-methoxybenzyl)-anilin sein kann;

oder ein pharmazeutisch annehmbares Salz davon.

2. Ein Verfahren für die Herstellung einer Verbindung der Strukturformel (I) nach Anspruch 1, umfassend

(a) das Behandeln eines entsprechend substituierten 2-Hydroxybenzaldehyds der Strukturformel (II)

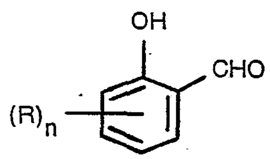

(II)

mit einem Amin der Formel $R^1NH_2$ unter Bildung einer Schiff'schen Base; und

(b) das Behandeln der Schiff'schen Base mit einem Reduktionsmittel,

sowie gegebenenfalls das Neutralisieren des erhaltenen Produktes unter Bildung des pharmazeutisch annehmbaren Salzes.

3. Eine pharmazeutische Zusammensetzung für die Behandlung topischer Entzündung, enthaltend einen pharmazeutischen Träger und eine wirksame Menge einer Verbindung der Formel (I) nach Anspruch 1, und/oder N-(2-Hydroxy-5-methoxybenzyl)-anilin.

4. Die pharmazeutische Zusammensetzung des Anspruchs 3, worin die Verbindung N-(2-Hydroxy-5-methoxybenzyl)-anilin ist.

**Patentanspruch für den Vertragsstaat: AT**

Ein Verfahren für die Herstellung einer Verbindung der Strukturformel (I)

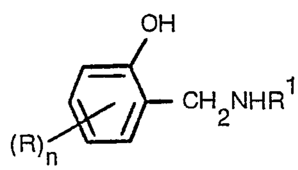

(I)

worin

R

(a) Fluor, mit der Ausnahme, daß, falls n 2 ist, die zwei Fluorgruppen nicht in Stellung 3 bzw. 5 vorliegen können;

(b) Methoxy, mit der Ausnahme, daß, falls n 1 ist, die Methoxygruppe nicht in Stellung 3 vorliegen kann; Ethoxy, n-Propoxy oder i-Propoxy;

(c) Methylthio, Ethylthio, n-Propylthio oder i-Propylthio;

(d) $—OCH_2—O—$;

(e) —COOH; oder

(f) Aryloxy bedeutet;

$R^1$

(a) unsubstituiertes oder substituiertes Phenyl, mit der Ausnahme, daß, falls R Fluor ist, $R^1$ nicht ein halogensubstituiertes Phenyl sein kann; oder

(b) unsubstituiertes oder substituiertes Heteroaryl bedeutet; und

n 1 oder 2 ist;

mit der Maßgabe, daß die Verbindung der Strukturformel (I) nicht N-(2-Hydroxy-5-methoxybenzyl)-anilin sein kann;

oder eines pharmazeutisch annehmbaren Salzes davon, umfassend:

(a) das Behandeln eines entsprechend substituierten 2-Hydroxybenzaldehyds der Strukturformel (II)

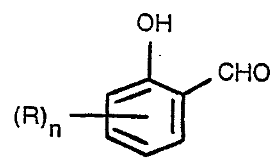

(II)

mit einem Amin der Formel $R^1NH_2$ unter Bildung einer Schiff'schen Base; und

(b) das Behandeln der Schiff'schen Base mit einem Reduktionsmittel,

sowie gegebenenfalls das Neutralisieren des erhaltenen Produktes unter Bildung des pharmazeutisch annehmbaren Salzes.

**Revendications pour les Etats contractants: BE CH DE FR GB IT LI LU NL SE**

1. Un composé de formule développée (I)

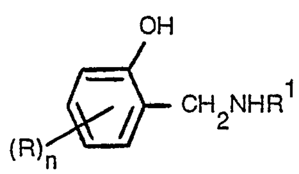

(I)

dans laquelle:

R est

(a) un fluoro, si ce n'est que lorsque n est 2, les deux radicaux fluoro ne peuvent pas être respectivement en les positions 3 et 5;

(b) un méthoxy, si ce n'est que lorsque n est 1, le radical méthoxy ne peut pas être en position 3; un éthoxy, un n-propoxy ou un isopropoxy;

(c) un méthylthio, un éthylthio, un n-propylthio ou un isopropylthio;

(d) $—OCH_2—O—$;

(e) —COOH; ou

(f) un aryloxy;

$R^1$ est

(a) un phényl non substitué ou substitué, si ce n'est que lorsque R est un fluoro, $R^1$ ne peut pas être un phényle halogéné-substitué; ou

(b) un hétéroaryle non substitué ou substitué;

n est 1 ou 2;

sous réserve que le composé de formule développée (I) ne peut pas être la N-(2-hydroxy-5-méthoxybenzyl)aniline;

ou un de ses sels acceptables en pharmacie.

2. Un procédé pour la préparation d'un composé de formule développée (I) selon la revendication 1, comprenant

(a) le traitement d'un 2-hydroxybenzaldéhyde convenablement substitué de formule développée (II)

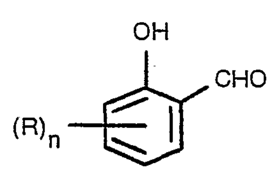

(II)

avec une amine de formule $R^1NH_2$ pour former une base de Schiff; et

(b) le traitement de la base de Schiff avec un agent réducteur,

et éventuellement, la neutralisation du produit obtenu pour former le sel acceptable en pharmacie.

3. Une composition pharmaceutique pour le traitement de l'inflammation locale comprenant un véhicule pharmaceutique et une quantité efficace d'un composé de formule (I) selon la revendication 1 et/ou de N-(2-hydroxy-5-méthoxybenzyl)aniline.

4. La composition pharmaceutique de la revendication 3, dans laquelle le composé est la N-(2-hydroxy-5-méthoxybenzyl)aniline.

**Revendication pour l'Etat contractant: AT**

Un procédé pour la préparation d'un composé de formule développée (I)

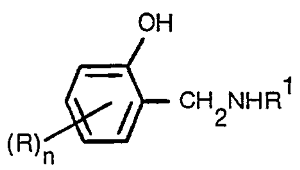

(I)

dans laquelle:

R est

(a) un fluoro, si ce n'est que lorsque n est 2, les deux radicaux fluoro ne peuvent pas être respectivement en les positions 3 et 5;

(b) un méthoxy, si ce n'est que lorsque n est 1, le radical méthoxy ne peut pas être en position 3; un éthoxy, un n-propoxy ou un iso-propoxy;

(c) un méthylthio, un éthylthio, un n-propylthio ou un isopropylthio;

(d) $—OCH_2—O—$;

(e) —COOH; ou

(f) un aryloxy;

$R^1$ est

(a) un phényl non substitué ou substitué, si ce n'est que lorsque R est un fluoro, $R^1$ ne peut pas être un phényle halogéné-substitué; ou

(b) un hétéroaryle non substitué ou substitué;

n est 1 ou 2;

sous réserve que le composé de formule développée (I) ne peut pas être la N-(2-hydroxy-5-méthoxybenzyl)aniline;

et le sel acceptable en pharmacie correspondant, comprenant:

(a) le traitement d'un 2-hydroxybenzaldéhyde convenablement substitué de formule développée (II)

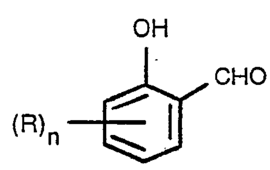

(II)

avec une amine de formule $R^1NH_2$ pour former une base de Schiff, et

(b) le traitement de la base de Schiff avec un agent réducteur;

et éventuellement, la neutralisation du produit obtenu pour former le sel acceptable en pharmacie.